(19) Europäisches Patentamt
European Patent Office
Office européen des brevets

(11) EP 1 312 346 B1

(12) **FASCICULE DE BREVET EUROPEEN**

(45) Date de publication et mention de la délivrance du brevet:
**07.04.2010 Bulletin 2010/14**

(51) Int Cl.:
***A61K 8/898** (2006.01)* ***A61Q 5/00** (2006.01)*

(21) Numéro de dépôt: **02292748.7**

(22) Date de dépôt: **04.11.2002**

(54) **Utilisation de silicones aminées particulières en post traitement de colorations directes ou d'oxydation de fibres kératiniques**

Verwendung von speziellen Aminosilikonen zur Nachbehandlung in der direkten oder oxidativen Färbung von Keratinfasern

Use or particular aminosilicones as a post-treatment in direct or oxidative dyeing of keratinous fibres

(84) Etats contractants désignés:
**AT BE BG CH CY CZ DE DK EE ES FI FR GB GR IE IT LI LU MC NL PT SE SK TR**

(30) Priorité: **08.11.2001 FR 0114475**

(43) Date de publication de la demande:
**21.05.2003 Bulletin 2003/21**

(73) Titulaire: **L'Oréal**
**75008 Paris (FR)**

(72) Inventeurs:
- **Gawtrey, Jonathan**
**92100 Boulogne (FR)**
- **Restle, Serge**
**95390 Saint-Prix (FR)**
- **Devin-Baudoin, Priscille**
**92170 Vanves (FR)**
- **Sabbagh, Anne**
**92500 Rueil Malmaison (FR)**

(74) Mandataire: **Fevrier, Murielle Françoise E.**
**L'Oréal**
**D.I.P.I.**
**25-29 Quai Aulagnier**
**92600 Asnières (FR)**

(56) Documents cités:
**EP-A- 0 890 355** **DE-A- 19 754 053**
**GB-A- 2 165 550**

Remarques:
Le dossier contient des informations techniques présentées postérieurement au dépôt de la demande et ne figurant pas dans le présent fascicule.

EP 1 312 346 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention a pour objet l'utilisation, en post traitement d'une coloration directe ou d'oxydation des fibres kératiniques humaines et plus particulièrement des cheveux, d'une composition comprenant au moins une silicone aminée particulière.
Elle a aussi pour objet un procédé de coloration directe ou d'oxydation des fibres kératiniques humaines et plus particulièrement des cheveux comprenant un post traitement avec une composition comprenant au moins une silicone aminée particulière.

Il existe principalement deux types de coloration des fibres kératiniques. La coloration directe, mettant en oeuvre en présence ou en l'absence d'agents oxydants, des colorants directs et / ou des pigments qui sont des molécules colorées, conférant aux fibres une couleur temporaire qui s'estompe après quelques shampooings, et la coloration dite "coloration d'oxydation" mettant en oeuvre des précurseurs de colorants d'oxydation et un agent oxydant, qui confère aux fibres une couleur plus tenace que la précédente.
L'utilisation d'un agent oxydant entraîne en général une certaine dégradation de la fibre kératinique.

Le document EP 890335 décrit une composition cosmétique pour la coloration d'oxydation des cheveux comprenant une silicone aminée qui peut être substituée par un radical alcoxy.

On constate actuellement une tendance très nette à l'augmentation de la fréquence des shampooings, ce qui se traduit par une dégradation plus importante des colorations entre deux applications.

Il existe donc un besoin d'améliorer la ténacité des colorations directes ou d'oxydation, en particulier vis à vis des shampooings.

Après d'importantes études sur la question, la demanderesse a découvert de manière tout à fait inattendue et surprenante que l'utilisation, en post traitement sur des fibres kératiniques humaines et plus particulièrement des cheveux, d'une composition comprenant au moins une silicone aminée particulière, permettait de résoudre ce problème.
Cette découverte est à l'origine de la présente invention.

En outre, ce post traitement améliore l'état de la fibre, en particulier dans le cas d'une coloration préalable en présence d'agent oxydant.
Par amélioration de l'état de la fibre on entend une diminution de la porosité ou de la solubilité alcaline de la fibre et une amélioration des propriétés cosmétiques et en particulier du lissage, de la douceur et de la facilité de démêlage et de coiffage.

Cet effet est rémanent, c'est à dire durable.
La porosité se mesure par la fixation à 37°C et à pH10, en 2 minutes, de la 2-nitro paraphénylènediamine à 0,25% dans un mélange éthanol / tampon pH 10 (rapport volumique 10/90).
La solubilité alcaline correspond à la perte de masse d'un échantillon de 100 mg de fibres kératiniques sous l'action de la soude décinormale pendant 30 minutes à 65°C.

Un premier objet de l'invention concerne donc l'utilisation, en post traitement d'une coloration d'oxydation ou d'une coloration directe des fibres kératiniques humaines et plus particulièrement des cheveux, d'une composition comprenant au moins une silicone aminée de formules (I) ou (II) décrites ci-après.

Ladite utilisation a notamment pour objet d'améliorer la ténacité aux shampooings des dites colorations et/ou l'état de la fibre après coloration, notamment dans le cas de coloration avec oxydant. Ce post traitement peut se faire immédiatement après teinture et un éventuel rinçage, ou de manière différée, et de manière unique ou répétitive entre deux colorations.

Un second objet de l'invention concerne un procédé de coloration consistant à appliquer sur les fibres kératiniques humaines et plus particulièrement des cheveux, une composition colorante directe ou d'oxydation pendant un temps suffisant pour développer la couleur, et de faire suivre cette application, après rinçage ou non rinçage, séchage ou non séchage, par l'application d'une composition comprenant au moins une silicone aminée de formules (I) ou (II).

<u>Silicones aminées</u>

Les silicones aminées de formules (I) ou (II) selon l'invention sont les suivantes :

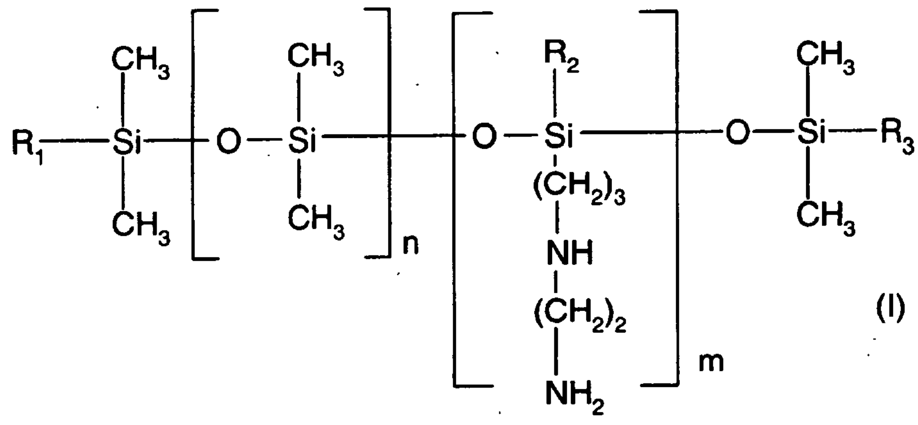

(I)

dans laquelle :

m et n sont des nombres tels que la somme (n + m) peut varier notamment de 1 à 1 000 et en particulier de 50 à 250 et plus particulièrement de 100 à 200,
n pouvant désigner un nombre de 0 à 999 et notamment de 49 à 249 et plus particulièrement de 125 à 175 et m pouvant désigner un nombre de 1 à 1 000, et notamment de 1 à 10 et plus particulièrement de 1 à 5;
$R_1$, $R_2$, $R_3$, identiques ou différents, représentent un radical hydroxy ou alcoxy en $C_1$-$C_4$, l'un au moins des radicaux $R_1$ à $R_3$ désignant un radical alcoxy.

De préférence le radical alcoxy est un radical méthoxy.
Le rapport molaire Hydroxy/Alcoxy est de préférence compris entre 0,2:1 et 0,4:1 et de préférence entre 0,25:1 et 0,35:1 et plus particulièrement est égal à 0,3.
La silicone aminée de formule (I) présente une masse moléculaire moyenne en poids allant de préférence de 2000 à 1000000 et encore plus particulièrement de 3500 à 200000.

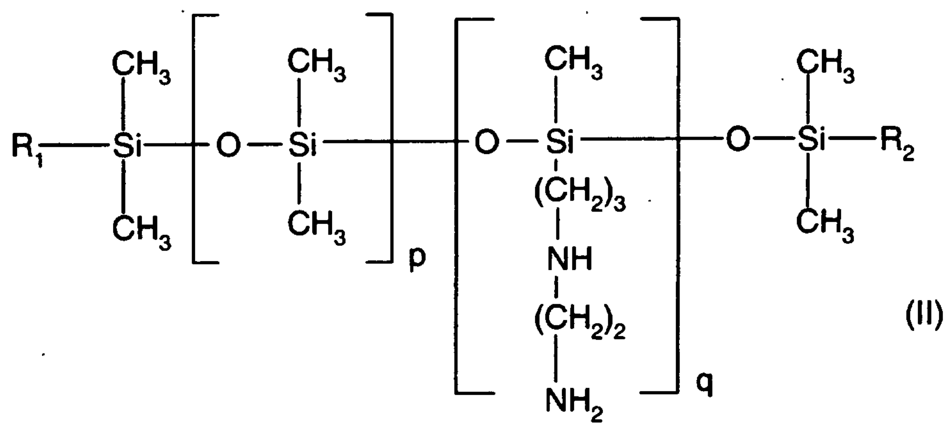

(II)

dans laquelle :

p et q sont des nombres tels que la somme (p + q) peut varier notamment de 1 à 1 000 et en particulier de 50 à 350, et plus particulièrement de 150 à 250
p pouvant désigner un nombre de 0 à 999 et notamment de 49 à 349 et plus particulièrement de 159 à 239 et q pouvant désigner un nombre de 1 à 1 000, et notamment de 1 à 10 et plus particulièrement de 1 à 5;
$R_1$, $R_2$, différents, représentent un radical hydroxy ou alcoxy en $C_1$-$C_4$, l'un au moins des radicaux $R_1$ à $R_2$ désignant un radical alcoxy.

De préférence le radical alcoxy est un radical méthoxy.
Le rapport molaire Hydroxy/Alcoxy est de préférence compris entre 1:0,8 et 1:1,1 et de préférence entre 1:0,9

et 1:1 et plus particulièrement est égal à 1:0,95.

La silicone aminée de formule (II) présente une masse moléculaire moyenne en poids allant de préférence de 2000 à 200.000, et encore plus particulièrement de 5.000 à 100.000 et plus particulièrement encore de 10.000 à 50.000.

Les masses moléculaires moyennes en poids de ces silicones aminées sont mesurées par Chromatographie par Perméation de Gel (GPC) à température ambiante en équivalent polystyrène. Les colonnes utilisées sont des colonnes μ styragel. L'éluant est le THF, le débit est de 1 ml/mn. On injecte 200 μl d'une solution à 0,5% en poids de silicone dans le THF. La détection se fait par réfractométrie et UVmétrie.

Les produits commerciaux correspondant à ces silicones de structure (I) ou (II) peuvent inclure dans leur composition une ou plusieurs autres silicones aminées dont la structure est différente des structures (I) et (II).

Un produit contenant des silicones aminées de structure (I) est proposé par la société WACKER sous la dénomination BELSIL ADM 652®.

Des produits contenant des silicones aminées de structure (II) sont proposés par la société WACKER sous la dénomination Fluid WR 1300®, et BELSIL ADM 6057®.

Lorsque ces silicones aminées sont mises en oeuvre, une forme de réalisation particulièrement intéressante est leur utilisation sous forme d'émulsion huile-dans-eau. L'émulsion huile-dans-eau peut comprendre un ou plusieurs tensioactifs. Les tensioactifs peuvent être de toute nature mais de préférence cationique et/ou non ionique.

Les particules de silicone dans l'émulsion ont une taille moyenne allant généralement de 3 nm à 500 nanomètres.

De préférence, notamment pour les silicones aminées de formule (II), on utilise des microémulsions de taille allant de 5 nm à 60 nanomètres et plus particulièrement de 10 nm à 50 nanomètres.

On peut utiliser selon l'invention les microémulsions de silicones aminées de formule (II) proposées sous la dénomination FINISH CT 96 E® ou SLM 28020® par la société WACKER.

De préférence la silicone aminée de formule (I) ou (II) est choisie de telle façon que l'angle de contact avec l'eau d'un cheveu traité avec une composition contenant 2% MA (matières actives) de ladite silicone selon l'invention soit compris entre 90 et 180° et de préférence entre 90 et 130°, bornes incluses.

De préférence la composition contenant la ou les silicones aminées de formule (I) ou (II) est telle que l'angle de contact d'un cheveu traité avec la dite composition est compris entre 90 et 180° et de préférence entre 90 et 130°, bornes incluses.

La mesure de l'angle de contact est basée sur l'immersion d'un cheveu dans de l'eau distillée. Il consiste à évaluer la force exercée par l'eau sur le cheveu lors de son immersion de l'eau distillée et lors de son retrait. Les forces ainsi mesurées sont directement reliées à l'angle de contact θ entre l'eau et la surface du cheveu. Le cheveu est dit hydrophile lorsque l'angle θ est compris entre 0 et 90°, hydrophobe lorsque cet angle est compris entre 90° et 180°, bornes incluses.

Le test s'effectue avec des mèches de cheveux naturels ayant été décolorés dans les mêmes conditions puis lavés.

Chaque mèche de 1 g est placée dans un cristallisoir de 75 mm de diamètre puis recouverte de façon homogène par 5 mL de la formule à tester. La mèche est ainsi laissée 15 minutes à température ambiante puis rincée pendant 30 secondes. La mèche essorée est laissée à l'air libre jusqu'à ce qu'elle soit complètement sèche.

Pour chaque évaluation, 10 cheveux ayant subi le même traitement sont analysés. Chaque échantillon, fixé à une microbalance de précision, est immergé par la pointe dans un récipient rempli d'eau distillée. Cette balance DCA (« Dynamic Contact Angle Analyser »), de la société CAHN Instruments, permet de mesurer la force (F) exercée par l'eau sur le cheveu.

Parallèlement, le périmètre du cheveu (P) est mesuré via une observation microscopique.

La force moyenne de mouillabilité sur 10 cheveux et la section des cheveux analysés permettent d'obtenir l'angle de contact du cheveu sur l'eau, selon la formule :

$$F = P * \Gamma lv * \cos\theta$$

où F est la force de mouillabilité exprimée en Newton, P le périmètre du cheveu en mètre, $\Gamma lv$ la tension interfaciale liquide / vapeur de l'eau en $J/m^2$ et θ l'angle de contact.

Le produit SLM 28020® de WACKER à 12% dans l'eau (soit 2% en silicone aminée) conduit à un angle de contact de 93° selon le test indiqué ci-dessus.

La silicone aminée est utilisée de préférence dans la composition de post traitement en une quantité allant de 0,01 à 20% en poids du poids total de la composition. Plus préférentiellement, cette quantité va de 0,1 à 15% en poids et encore plus particulièrement de 0,5 à 10 % en poids.

La composition de post traitement peut contenir tous les ingrédients classiquement utilisés en cosmétique et en particulier dans le domaine capillaire. En particulier elle peut contenir des tensioactifs et/ou des polymères additionnels. Ces tensioactifs et ces polymères peuvent être de nature non-ionique, cationique, anionique ou amphotère.

Parmi les polymères additionnels, les silicones aminées autres que celles de l'invention sont particulièrement préférées.

La composition de post traitement présente un pH allant de 2 à 11 et de préférence de 4 à 9.

Elle peut se présenter sous diverses formes telles que lotions, gels, crèmes, shampooings, sticks, mousses, sprays. Pour certaines de ces formes, elle peut être conditionnée en flacon pompe ou dans un récipient aérosol. Dans le cas de l'aérosol, la composition est associée à un agent propulseur qui peut être par exemple un alcane ou un mélange d'alcane, du diméthyl éther, de l'azote, du protoxyde d'azote, du gaz carbonique ou des halgénoalcanes, ainsi que leurs mélanges.

Une forme particulièrement préférée selon l'invention est la forme shampooing.

Dans ce cas, la composition contient au moins un agent tensioactif qui est de préférence anionique. De préférence alors, elle contient un mélange de tensioactifs dont au moins un agent tensioactif anionique, le ou les autres tensioactifs étant préférentiellement non ioniques ou amphotères.

Comme il est indiqué ci dessus, la composition de post traitement peut être appliquée immédiatement après coloration ou de manière différée. Par différée, on entend une application se faisant quelques heures, un jour ou plusieurs jours (de 1 à 60 jours) après la coloration.

De préférence, plusieurs applications sont effectuées entre deux colorations.

Le nombre d'applications entre deux colorations est de préférence compris entre 1 et 60 et encore plus préférentiellement entre 2 et 30.

La composition de post traitement peut être utilisée en mode rincé ou en mode non-rincé, c'est à dire que son application est suivie ou non d'un rinçage.

Dans le premier cas, le temps de pose de la composition de post traitement est compris entre quelques secondes et 60 minutes et de préférence entre 30 secondes et 15 minutes.

La température d'application de la composition de post traitement peut varier de 10°C à 70°C. De préférence l'application s'effectuera entre 10 et 60°C et plus particulièrement à la température ambiante.

La nature et la concentration des colorants présents dans les compositions colorantes n'est pas critique.

Dans le cas des colorations directes ( en présence ou en l'absence d'agents oxydants) les compositions colorantes comprennent au moins un colorant choisi parmi les colorants directs nitrés benzéniques neutres, acides ou cationiques, les colorants directs azoïques

ou méthiniques neutres, acides ou cationiques, les colorants directs quinoniques et en particulier anthraquinoniques neutres, acides ou cationiques, les colorants directs aziniques, les colorants directs triarylméthaniques, les colorants directs indoaminiques, les colorants directs naturels ou leurs mélanges.

Dans le cas des colorations d'oxydation, les compositions colorantes comprennent au moins une base d'oxydation.

Les bases d'oxydation sont choisies parmi celles classiquement utilisées en teinture d'oxydation, et parmi lesquelles on peut notamment citer les ortho- et paraphénylènediamines, les bases doubles, les ortho- et para- aminophénols, les bases hétérocycliques ainsi que leurs sels d'addition avec un acide .

Généralement, les compositions colorantes d'oxydation comprennent un ou plusieurs coupleurs.

Les coupleurs utilisables sont ceux classiquement utilisés dans les compositions de teinture d'oxydation, c'est-à-dire des métaphénylènediamines, des métaaminophénols et des métadiphénols, les dérivés mono- ou poly-hydroxylés du naphtalène, le sésamol et ses dérivés et des composés hétérocycliques tels que par exemple les coupleurs indoliques, les coupleurs indoliniques, les coupleurs pyridiniques et leurs sels d'addition avec un acide.

La nature de l'agent oxydant utilisé dans la coloration directe éclaircissante (coloration directe avec un agent oxydant) ou dans la coloration d'oxydation n'est pas critique.

L'agent oxydant est de préférence choisi dans le groupe formé par le peroxyde d'hydrogène, le peroxyde d'urée, les bromates ou ferricyanures de métaux alcalins, les persels tels que les perborates et les persulfates. On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Les exemples suivants sont destinés à illustrer l'invention. Celle-ci n'est cependant pas limitée à ces modes de réalisation.

<u>EXEMPLES</u>

On a préparé les cinq compositions de post traitement A, B, C, D, E suivantes.

(exprimées en grammes de Matière Active (MA)

| **Composition A** | |
|---|---|
| Polydiméthylsiloxane de formule (I) selon l'invention proposé sous la dénomination BELSIL ADM 652® par la société WACKER | 2,0 |

(suite)

| Composition A | |
|---|---|
| Solvant q.s.p | 100 |

| Composition B | |
|---|---|
| Polydiméthylsiloxane de formule (II) selon l'invention proposé sous la dénomination BELSIL ADM 6057® par la société WACKER | 2,0 |
| Eau déminéralisée q.s.p | 100 |

| Composition C | |
|---|---|
| Polydiméthylsiloxane de formule (II) selon l'invention proposé sous la dénomination SLM 28020® par la société WACKER | 2 |
| Eau déminéralisée q.s.p | 100 |

| Composition D | |
|---|---|
| Lauryléthersulfate de sodium à 2,2 moles d'oxyde d'éthylène | 7 |
| Cocoyl bétaïne | 2,5 |
| Distéarate de glycol | 1,5 |
| Polydiméthylsiloxane de formule (II) selon l'invention proposé sous la dénomination SLM 28020® par la société WACKER | 1,5 |
| Hydroxyéthylcellulose quaternisé par le chlorure de 2,3-époxy-propyltriméthylammonium vendu sous la marque Ucare Polymer JR-400® par Union Carbide | 0,4 |
| Polymère acrylique en émulsion vendu sous la marque Aqua SF1® par Noveon | 0,8 |
| Conservateurs | q.s. |
| Agents de pH. q.s.p | pH 5 |
| Eau déminéralisée q.s.p | 100 |

| Composition E | |
|---|---|
| Lauryléthersulfate de sodium à 2,2 moles d'oxyde d'éthylène | 7 |
| Cocoyl bétaïne | 2,5 |
| Distéarate de glycol | 1,5 |
| Polydiméthylsiloxane viscosité 60000 mm2/s vendu sous la dénomination Dow Corning 200 Fluid® par DOW CORNING | 1,0 |
| Polydiméthylsiloxane de formule (I) selon l'invention proposé sous la dénomination BELSIL ADM 652® par la société WACKER | 2 |
| Hydroxyéthylcellulose quaternisé par le chlorure de 2,3-époxy-propyltriméthylammonium vendu sous la marque Ucare Polymer JR-400® par Union Carbide | 0,4 |
| Polymère acrylique en émulsion vendu sous la marque Aqua SF1® par Noveon | 0,8 |
| Conservateurs | q.s. |

(suite)

| Composition E | | |
|---|---|---|
| Agents de pH | q.s.p | pH 5 |
| Eau déminéralisée | q.s.p | 100 |

Les compositions A et B ont été appliquées pendant 2 minutes sur des mèches de cheveux modérément décolorées et colorées par la coloration d'oxydation du commerce MAJIROUGE®, nuance 7,40.
Après rinçage et séchage, on a effectué sur ces mèches 5 shampoings standard.
Pour comparaison, des mèches colorées dans les mêmes conditions, et ayant subi le même protocole de shampooings ont subi un post-traitement à l'eau déminéralisée au lieu du post-traitement avec les compositions A et B .
Résultats : la dégradation de la couleur (DE dans le système L*a*b*) par rapport à une mèche non shampooinée a été évaluée comparativement au cas où on a remplacé le post traitement selon l'invention par un post traitement à l'eau déminéralisée.
On a obtenu les résultats suivants:

| | |
|---|---|
| Composition A | DE=6,0 |
| Composition B | DE=5,7 |
| Placebo | DE=8,0 |

Plus le DE est faible, moindre est la dégradation. Les compositions A et B en post traitement de la coloration d'oxydation MAJIROUGE® 7,40 ont donc protégé de la dégradation aux shampooings.
Par ailleurs l'état des fibres capillaires a été satisfaisant.
La composition C a été appliquée sans rinçage ultérieur sur des mèches de cheveux naturels à 90% de blancs colorées par la coloration d'oxydation MAJIROUGE® nuance 6,64 . Après séchage, on a effectué 8 shampoings DOP.
Résultats : la dégradation de la couleur (DE dans le système L*a*b*) par rapport à une mèche non shampooinée a été évaluée comparativement au cas où on n'a pas effectué le post traitement selon l'invention.
On a obtenu les résultats suivants :

| | |
|---|---|
| Composition C | DE=4,6 |
| Placebo | DE=7,3 |

Plus le DE est faible, moindre est la dégradation. La composition C en post traitement de la coloration d'oxydation MAJIROUGE® 6,64 a donc protégé de la dégradation aux shampooings.
Par ailleurs l'état de la fibre capillaire a été satisfaisant.
Les compositions D et E ont été utilisées comme shampooings d'entretien sur des colorations d'oxydation ou des colorations directes. Les dégradations des nuances initiales ont été moindres que dans le cas où on a utilisé un shampooing d'entretien standard ( sans la silicone aminée de l'invention). L'état des fibres capillaires a été jugé meilleur.

## Revendications

1. Utilisation en post traitement d'une coloration directe ou d'oxydation des fibres kératiniques humaines d'une composition comprenant au moins une silicone aminée de formules (I) ou (II) suivantes :

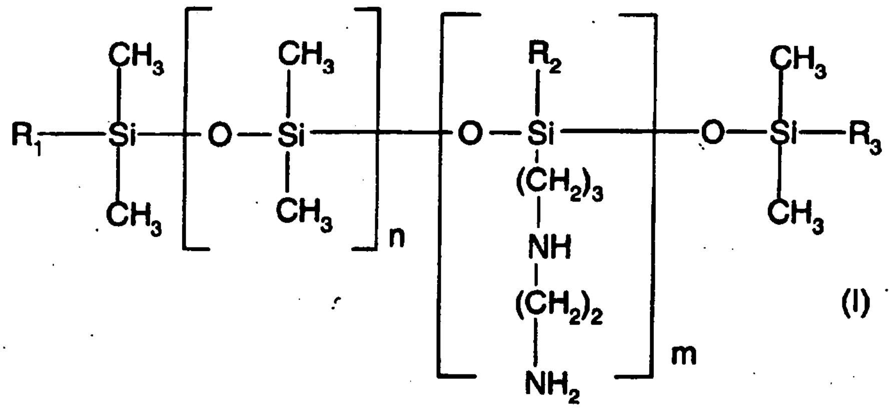

(I)

formule (I) dans laquelle :
m et n sont des nombres tels que la somme (n + m) varie de 1 à 1 000,
n désignant un nombre de 0 à 999 et m désignant un nombre de 1 à 1 000;
$R_1$, $R_2$, $R_3$, identiques ou différents, représentent un radical hydroxy ou alcoxy en $C_1$-$C_4$ ; l'un au moins des radicaux $R_1$ à $R_3$ désignant un radical alcoxy ;

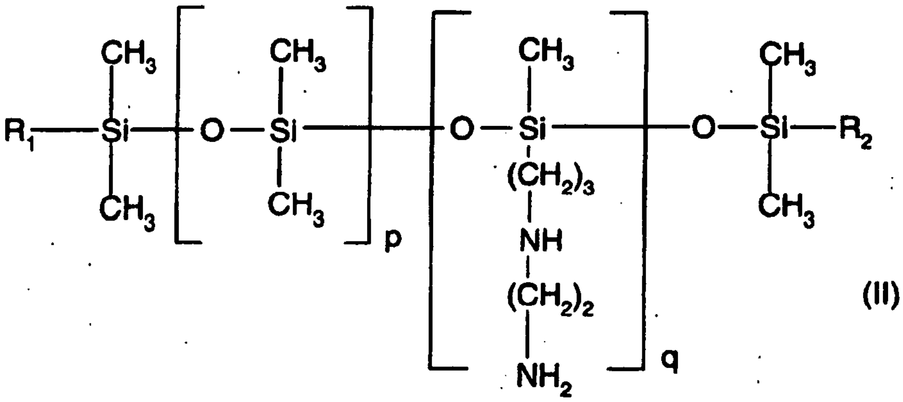

(II)

formule (II) dans laquelle:
p et q sont des nombres tels que la somme (p + q) varie de 1 à 1 000,
p désignant un nombre de 0 à 999 et q désignant un nombre de 1 à 1 000;
$R_1$, $R_2$, différents, représentent un radical hydroxy ou alcoxy en $C_1$-$C_4$, l'un au moins des radicaux $R_1$ à $R_2$ désignant un radical alcoxy.

2. Utilisation selon la revendication 1, **caractérisée par le fait que** le radical alcoxy $C_1$-$C_4$ désigne le radical méthoxy.

3. Utilisation selon l'une quelconque des revendications précédentes, **caractérisée par le fait que** pour les silicones aminées de formule (I), le rapport molaire Hydroxy/Alcoxy est compris entre 0,2:1 et 0,4:1.

4. Utilisation selon l'une quelconque des revendications 1 à 2, **caractérisée par le fait que** pour les silicones aminées de formule (II), le rapport molaire Hydroxy/Alcoxy est compris entre 1:0,8 et 1:1,1.

5. Utilisation selon l'une quelconque des revendications 1 à 3, **caractérisée par le fait que** la silicone aminée de formule (I) présente une masse moléculaire moyenne en poids allant de 2000 à 1000000.

6. Utilisation selon l'une quelconque des revendications 1 à 2 et 4, **caractérisée par le fait que** la silicone aminée de formule (II) présente une masse moléculaire moyenne en poids allant de 2000 à 200.000.

7. Utilisation selon l'une quelconque des revendications précédentes, **caractérisée par le fait que** la silicone aminée de formule (I) ou (II) est sous forme d'émulsion huile-dans-eau comprenant des agents tensioactifs.

8. Utilisation selon la revendication 7, **caractérisée par le fait que** l'émulsion comprend au moins un agent tensioactif cationique et/ou non ionique.

9. Utilisation selon l'une quelconque des revendications 7 ou 8, **caractérisée par le fait que** les particules de silicone dans l'émulsion ont une taille allant de 3 nm à 500 nanomètres.

10. Utilisation selon l'une quelconque des revendications précédentes, **caractérisée par le fait que** la ou les silicones aminées de formules (I) ou (II) sont présentes dans la composition de post traitement en une quantité allant de 0,01 à 20% en poids du poids total de la composition.

11. Utilisation selon la revendication 10, **caractérisée par le fait que** la ou les silicones aminées de formule (I) ou (II) sont présentes dans la composition de post traitement en une quantité allant de 0,1 à 15% en poids du poids total de la composition.

12. Utilisation selon la revendication 11, **caractérisée par le fait que** la ou les silicones aminées de formule (I) ou (II) sont présentes dans la composition de post traitement en une quantité allant de 0,5 à 10 % en poids du poids total de la composition.

13. Utilisation selon l'une quelconque des revendications précédentes, **caractérisée par le fait que** la composition de post-traitement se présente sous forme de lotions, gels, crèmes, shampooings, sticks, mousses, sprays.

14. Utilisation selon l'une quelconque des revendications précédentes, **caractérisée par le fait que** la composition de post-traitement est conditionnée en flacon pompe ou dans un récipient aérosol.

15. Utilisation selon la revendication 14, **caractérisée par le fait que** la composition de post-traitement comprend au moins un agent propulseur choisi dans le groupe formé par les alcanes, le diméthyl éther, l'azote, le protoxyde d'azote, le gaz carbonique ou les halogénoalcanes.

16. Utilisation selon l'une quelconque des revendications précédentes, **caractérisée par le fait que** la composition de post-traitement comprend au moins un agent tensioactif de nature non-ionique, cationique, anionique ou amphotère.

17. Utilisation selon la revendication 16, **caractérisée par le fait que** la composition de post-traitement comprend un mélange d'agents tensioactifs comprenant au moins un agent tensioactif anionique, le ou les autres agents tensioactifs étant non ioniques ou amphotères.

18. Utilisation selon l'une quelconque des revendications précédentes, **caractérisée par le fait que** la composition de post-traitement comprend au moins un polymère additionnel autre que les silicones de formules (I) ou (II).

19. Utilisation selon la revendication 18, **caractérisée par le fait que** le polymère est de nature non-ionique, cationique, anionique ou amphotère.

20. Utilisation selon la revendication 19, **caractérisée par le fait que** le polymère est une silicone aminée différente des silicones de formules (I) ou (II).

21. Utilisation selon l'une quelconque des revendications précédentes, **caractérisée par le fait que** la composition de post-traitement présente un pH allant de 2 à 11.

22. Utilisation selon la revendication 1 pour améliorer la ténacité des colorations.

23. Utilisation selon la revendication 1 pour améliorer l'état des fibres kératiniques humaines.

24. Utilisation selon la revendication 23 pour améliorer l'état des fibres après une coloration comprenant un agent

oxydant.

**25.** Procédé de coloration des fibres kératiniques humaines **caractérisé par le fait qu'**il consiste à appliquer sur les fibres, dans une première étape, une composition colorante directe, à la laisser agir pendant un temps suffisant pour développer la couleur, puis, après avoir rincé ou non rincé les fibres et les avoir séchées ou non séchées, dans une seconde étape, appliquer une composition de post-traitement comprenant au moins une silicone de formule (I) ou (II) et telle que décrite à l'une quelconque des revendications 1 à 9, ladite composition de post-traitement étant appliquée, soit immédiatement, soit en différé, et les applications de ladite composition pouvant être répétées entre deux colorations.

**26.** Procédé de coloration des fibres kératiniques humaines **caractérisé par le fait qu'**il consiste à appliquer sur les fibres, dans une première étape, une composition colorante d'oxydation, à la laisser agir pendant un temps suffisant pour développer la couleur, puis, après avoir rincé ou non rincé les fibres et les avoir séchées ou non séchées, dans une second étape, appliquer une composition de post-traitement comprenant au moins une silicone de formule (I) ou (II) et telle que décrite à l'une quelconque des revendications 1 à 9, ladite composition de post-traitement étant appliquée, soit immédiatement, soit en différé, et les applications de ladite composition pouvant être répétées entre deux colorations.

**27.** Procédé selon l'une quelconque des revendications 25 ou 26, **caractérisé par le fait que** la composition de post-traitement est posée pendant un temps allant de quelques secondes à 60 minutes.

## Claims

**1.** Use, as a post-treatment of a process for colouring human keratin fibres with direct dyes or with oxidation dyes, of a composition comprising at least one aminosilicone of formula (I) or (II) below:

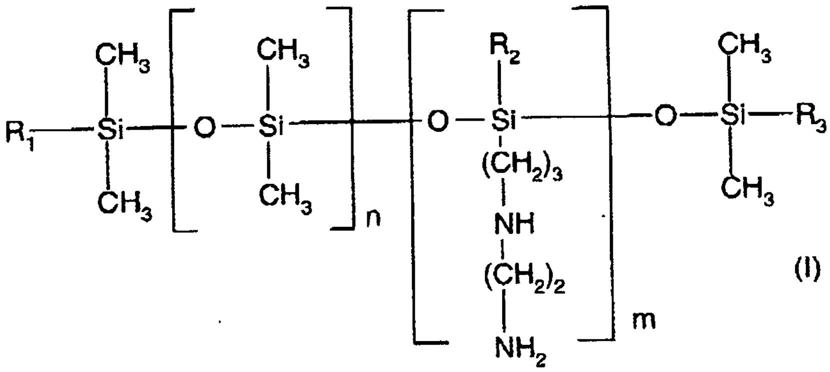

(I)

in which formula (I):
m and n are numbers such that the sum (n + m) ranges from 1 to 1000,
n denoting a number from 0 to 999 and m denoting a number from 1 to 1000;
$R_1$, $R_2$ and $R_3$, which may be identical or different, represent a hydroxyl or C1-C4 alkoxy radical, at least one of the radicals $R_1$ to $R_3$ denoting an alkoxy radical;

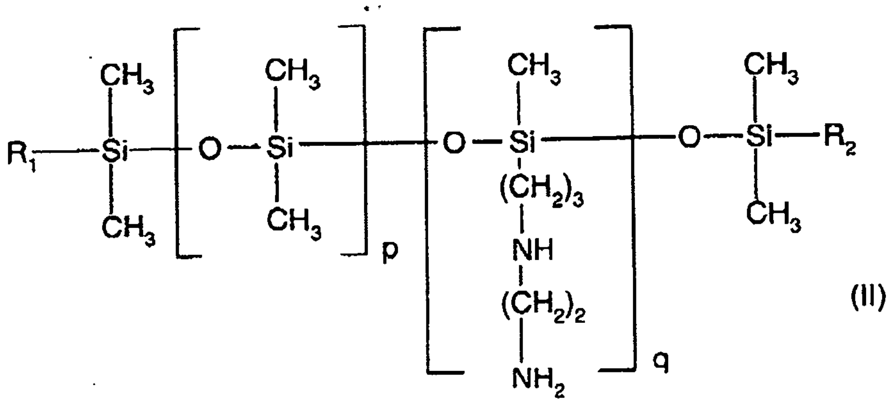

(II)

in which formula (II):
p and q are numbers such that the sum (p + q) ranges from 1 to 1000,
p denoting a number from 0 to 999 and q denoting a number from 1 to 1000;
$R_1$ and $R_2$, which are different, represent a hydroxyl or C1-C4 alkoxy radical, at least one of the radicals $R_1$ and $R_2$ denoting an alkoxy radical.

2. Use according to Claim 1, **characterized in that** the C1-C4 alkoxy radical denotes a methoxy radical.

3. Use according to either of the preceding claims, **characterized in that**, for the aminosilicones of formula (I), the hydroxyl/alkoxy molar ratio is between 0.2:1 and 0.4:1.

4. Use according to either of Claims 1 and 2, **characterized in that**, for the aminosilicones of formula (II), the hydroxyl/alkoxy molar ratio is between 1:0.8 and 1:1.1.

5. Use according to any one of Claims 1 to 3, **characterized in that** the aminosilicone of formula (I) has a weight-average molecular mass ranging from 2000 to 1 000 000.

6. Use according to any one of Claims 1, 2 and 4, **characterized in that** the aminosilicone of formula (II) has a weight-average molecular mass ranging from 2000 to 200 000.

7. Use according to any one of the preceding claims, **characterized in that** the aminosilicone of formula (I) or (II) is in the form of an oil-in-water emulsion comprising surfactants.

8. Use according to Claim 7, **characterized in that** the emulsion comprises at least one cationic and/or nonionic surfactant.

9. Use according to either of Claims 7 and 8, **characterized in that** the silicone particles in the emulsion range in size from 3 nm to 500 nanometres.

10. Use according to any one of the preceding claims, **characterized in that** the aminosilicone(s) of formula (I) or (II) is (are) present in the post-treatment composition in an amount ranging from 0.01% to 20% by weight relative to the total weight of the composition.

11. Use according to Claim 10, **characterized in that** the aminosilicone(s) of formula (I) or (II) is (are) present in the post-treatment composition in an amount ranging from 0.1% to 15% by weight relative to the total weight of the composition.

12. Use according to Claim 11, **characterized in that** the aminosilicone(s) of formula (I) or (II) is (are) present in the post-treatment composition in an amount ranging from 0.5% to 10% by weight relative to the total weight of the composition.

13. Use according to any one of the preceding claims, **characterized in that** the post-treatment composition is in the form of lotions, gels, creams, shampoos, sticks, mousses or sprays.

**14.** Use according to any one of the preceding claims, **characterized in that** the post-treatment composition is packaged in a pump-dispenser bottle or in an aerosol container.

**15.** Use according to Claim 14, **characterized in that** the post-treatment composition comprises at least one propellant chosen from the group formed by alkanes, dimethyl ether, nitrogen, nitrous oxide, carbon dioxide and haloalkanes.

**16.** Use according to any one of the preceding claims, **characterized in that** the post-treatment composition comprises at least one surfactant of nonionic, cationic, anionic or amphoteric nature.

**17.** Use according to Claim 16, **characterized in that** the post-treatment composition comprises a mixture of surfactants comprising at least one anionic surfactant, the other surfactant(s) being nonionic or amphoteric.

**18.** Use according to any one of the preceding claims, **characterized in that** the post-treatment composition comprises at least one additional polymer other than the silicones of formula (I) or (II).

**19.** Use according to Claim 18, **characterized in that** the polymer is of nonionic, cationic, anionic or amphoteric nature.

**20.** Use according to Claim 19, **characterized in that** the polymer is an aminosilicone different from the silicones of formula (I) or (II).

**21.** Use according to any one of the preceding claims, **characterized in that** the post-treatment composition has a pH ranging from 2 to 11.

**22.** Use according to Claim 1, for improving the resistance of colourations.

**23.** Use according to Claim 1, for improving the condition of human keratin fibres.

**24.** Use according to Claim 23, for improving the condition of fibres after a colouration comprising an oxidizing agent.

**25.** Process for colouring human keratin fibres, **characterized in that** it consists in applying to the fibres, in a first step, a direct dye composition, in leaving the dye to act for a time that is sufficient to develop the colour, and then, after optionally rinsing the fibres and optionally drying them, in a second step, in applying a post-treatment composition comprising at least one silicone of formula (I) or (II) as described in any one of Claims 1 to 9, the said post-treatment composition being applied either immediately or after an interval, and the applications of the said composition possibly being repeated between two colourations.

**26.** Process for colouring human keratin fibres, **characterized in that** it consists in applying to the fibres, in a first step, an oxidation dye composition, in leaving the dye to act for a time that is sufficient to develop the colour, and then, after optionally rinsing the fibres and optionally drying them, in a second step, in applying a post-treatment composition comprising at least one silicone of formula (I) or (II) as described in any one of Claims 1 to 9, the said post-treatment composition being applied either immediately or after an interval, and the applications of the said composition possibly being repeated between two colourations.

**27.** Process according to either of Claims 25 and 26, **characterized in that** the post-treatment composition is left to act for a time ranging from a few seconds to 60 minutes.

### Patentansprüche

**1.** Verwendung einer Zusammensetzung, die mindestens ein aminiertes Silicon der folgenden Formeln (I) oder (II) enthält, für die Nachbehandlung bei einer Direktfärbung oder oxidativen Färbung von menschlichen Keratinfasern:

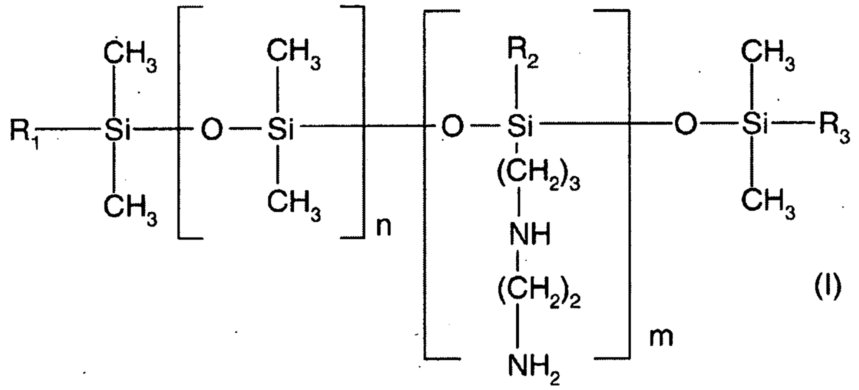

(I)

in der Formel (I):

m und n bedeuten Zahlen, die so gewählt sind, dass die Summe (n + m) im Bereich von 1 bis 1000 liegt,
n ist eine Zahl im Bereich von 0 bis 999 und m ist eine Zahl im Bereich von 1 bis 1000,
$R_1$, $R_2$ und $R_3$, die gleich oder verschieden sind, bedeuten eine Hydroxygruppe oder eine $C_{1-4}$-Alkoxygruppe, wobei mindestens eine der Gruppen $R_1$ bis $R_3$ eine Alkoxygruppe bedeutet;

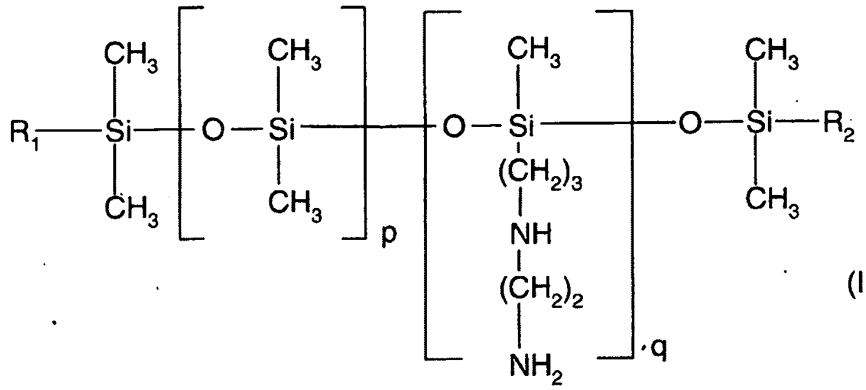

(II)

in der Formel (II):

p und q bedeuten Zahlen, die so gewählt sind, dass die Summe (p + q) im Bereich von 1 bis 1000 liegt,
p ist eine Zahl im Bereich von 0 bis 999 und q ist eine Zahl im Bereich von 1 bis 1000,
$R_1$ und $R_2$, die verschieden sind, bedeuten eine Hydroxygruppe oder eine $C_{1-4}$-Alkoxygruppe, wobei mindestens eine der Gruppen $R_1$ bis $R_2$ eine Alkoxygruppe bedeutet.

2. Verwendung nach Anspruch 1, **dadurch gekennzeichnet, dass** die $C_{1-4}$-Alkoxygruppe die Methoxygruppe bedeutet.

3. Verwendung nach einem der vorhergehenden Ansprüche, **dadurch gekennzeichnet, dass** für die aminierten Silicone der Formel (I) das Molverhältnis Hydroxy/Alkoxy im Bereich von 0,2:1 bis 0,4:1 liegt.

4. Verwendung nach einem der Ansprüche 1 bis 2, **dadurch gekennzeichnet, dass** für die aminierten Silicone der Formel (II) das Molverhältnis Hydroxy/Alkoxy im Bereich von 1:0,8 bis 1:1,1 liegt.

5. Verwendung nach einem der Ansprüche 1 bis 3, **dadurch gekennzeichnet, dass** das aminierte Silicon der Formel (I) eine gewichtmittlere Molmasse von 2.000 bis 1.000.000 aufweist.

6. Verwendung nach einem der Ansprüche 1 bis 2 und 4, **dadurch gekennzeichnet, dass** das aminierte Silicon der Formel (II) eine gewichtmittlere Molmasse im Bereich von 2.000 bis 200.000 aufweist.

7. Verwendung nach einem der vorhergehenden Ansprüche, **dadurch gekennzeichnet, dass** das aminierte Silicon der Formel (I) oder (II) in Form einer Öl-in-Wasser-Emulsion vorliegt, die grenzflächenaktive Stoffe enthält.

8. Verwendung nach Anspruch 7, **dadurch gekennzeichnet, dass** die Emulsion mindestens einen kationischen und/ oder nichtionischen grenzflächenaktiven Stoff enthält.

9. Verwendung nach einem der Ansprüche 7 oder 8, **dadurch gekennzeichnet, dass** die Siliconpartikel in der Emulsion eine Größe im Bereich von 3 bis 500 nm aufweisen.

10. Verwendung nach einem der vorhergehenden Ansprüche, **dadurch gekennzeichnet, dass** das oder die aminierte(n) Silicon(e) der Formel (I) oder (II) in der Zusammensetzung für die Nachbehandlung in einem Mengenanteil von 0,01 bis 20 Gew.-% des Gesamtgewichts der Zusammensetzung enthalten sind.

11. Verwendung nach Anspruch 10, **dadurch gekennzeichnet, dass** das oder die aminierte(n) Silicon(e) der Formel (I) oder (II) in der Zusammensetzung für die Nachbehandlung in einem Mengenanteil von 0,1 bis 15 Gew.-% des Gesamtgewichts der Zusammensetzung enthalten sind.

12. Verwendung nach Anspruch 11, **dadurch gekennzeichnet, dass** das oder die aminierte(n) Silicon(e) der Formel (I) oder (II) in der Zusammensetzung für die Nachbehandlung in einem Mengenanteil von 0,5 bis 10 Gew.-% des Gesamtgewichts der Zusammensetzung enthalten sind.

13. Verwendung nach einem der vorhergehenden Ansprüche, **dadurch gekennzeichnet, dass** die Zusammensetzung für die Nachbehandlung als Lotion, Gel, Creme, Haarwaschmittel, Stift, Schaum oder Spray vorliegt.

14. Verwendung nach einem der vorhergehenden Ansprüche, **dadurch gekennzeichnet, dass** die Zusammensetzung für die Nachbehandlung in einem Pumpflakon oder in einem Aerosolbehälter konfektioniert ist.

15. Verwendung nach Anspruch 14, **dadurch gekennzeichnet, dass** die Zusammensetzung für die Nachbehandlung mindestens ein Treibmittel enthält, das unter den Alkanen, Dimethylether, Stickstoff, Distickstoffoxid, Kohlendioxid oder Halogenalkanen ausgewählt ist.

16. Verwendung nach einem der vorhergehenden Ansprüche, **dadurch gekennzeichnet, dass** die Zusammensetzung für die Nachbehandlung mindestens einen grenzflächenaktiven Stoff vom nichtionischen, kationischen, anionischen oder amphoteren Typ enthält.

17. Verwendung nach Anspruch 16, **dadurch gekennzeichnet, dass** die Zusammensetzung für die Nachbehandlung ein Gemisch von grenzflächenaktiven Stoffen enthält, das mindestens einen anionischen grenzflächenaktiven Stoff umfasst, wobei der oder die anderen grenzflächenaktiven Stoffe nichtionisch oder amphoter sind.

18. Verwendung nach einem der vorhergehenden Ansprüche, **dadurch gekennzeichnet, dass** die Zusammensetzung für die Nachbehandlung mindestens ein ergänzendes Polymer enthält, das von den Siliconen der Formel (I) oder (II) verschieden ist.

19. Verwendung nach Anspruch 18, **dadurch gekennzeichnet, dass** das Polymer vom nichtionischen, kationischen, anionischen oder amphoteren Typ ist.

20. Verwendung nach Anspruch 19, **dadurch gekennzeichnet, dass** das Polymer ein aminiertes Silicon ist, das von den Siliconen der Formel (I) oder (II) verschieden ist.

**21.** Verwendung nach einem der vorhergehenden Ansprüche,
**dadurch gekennzeichnet, dass** die Zusammensetzung für die Nachbehandlung einen pH-Wert im Bereich von 2 bis 11 aufweist.

**22.** Verwendung nach Anspruch 1, um die Beständigkeit der Färbungen zu verbessern.

**23.** Verwendung nach Anspruch 1, um den Zustand menschlicher Keratinfasern zu verbessern.

**24.** Verwendung nach Anspruch 23, um den Zustand der Fasern nach einer Färbung mit einem Oxidationsmittel zu verbessern.

**25.** Verfahren zum Färben menschlicher Keratinfasern, **dadurch gekennzeichnet, dass** es darin besteht, in einem ersten Schritt eine Zusammensetzung für die Direktfärbung auf die Fasern aufzutragen und während einer Zeitspanne einwirken zu lassen, die ausreichend ist, um die Farbe zu bilden, und dann, nachdem die Fasern gegebenenfalls gespült und gegebenenfalls getrocknet wurden, in einem zweiten Schritt eine Zusammensetzung für die Nachbehandlung, die mindestens ein Silicon der Formel (I) oder (II) enthält und wie sie in einem der Ansprüche 1 bis 9 beschrieben ist, aufzubringen, wobei die Zusammensetzung für die Nachbehandlung entweder sofort oder später aufgetragen wird und die Anwendungen dieser Zusammensetzung zwischen zwei Färbungen wiederholt werden können.

**26.** Verfahren zum Färben menschlicher Keratinfasern, **dadurch gekennzeichnet, dass** es darin besteht, in einem ersten Schritt eine Zusammensetzung für die oxidative Färbung auf die Fasern aufzutragen und während einer Zeitspanne einwirken zu lassen, die ausreichend ist, um die Farbe zu bilden, und dann, nachdem die Fasern gegebenenfalls gespült und gegebenenfalls getrocknet wurden, in einem zweiten Schritt eine Zusammensetzung für die Nachbehandlung aufzutragen, die mindestens ein Silicon der Formel (I) oder (II) enthält und wie sie in einem der Ansprüche 1 bis 9 beschrieben ist, wobei die Zusammensetzung für die Nachbehandlung entweder sofort oder später aufgetragen wird und die Anwendungen dieser Zusammensetzung zwischen zwei Färbungen wiederholt werden können.

**27.** Verfahren nach einem der Ansprüche 25 oder 26, **dadurch gekennzeichnet, dass** Zusammensetzung für die Nachbehandlung während einer Zeitspanne von einigen Sekunden bis 60 min einwirken gelassen wird.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 890335 A